# EUROPÄISCHE PATENTSCHRIFT

(11) **EP 0 437 185 B1**
(45) Veröffentlichungstag und Bekanntmachung des Hinweises auf die Patenterteilung: **18.10.1995**
(21) Anmeldenummer: 90811026.5
(22) Anmeldetag: 21.12.1990
(51) Int. Cl.: C07D 405/06, B41M 5/145, B41M 5/30

(54) **Pyrrolin- bzw. indolinhaltige Phthalide**
Pyrroline-resp. indoline containing phthalides
Phtalide contenant une pyrroline, respectivement une indoline

(30) Priorität: 11.01.1990 CH 84/90
(43) Veröffentlichungstag der Anmeldung: 17.07.1991
(73) Patentinhaber: CIBA-GEIGY AG, 4002 Basel (CH)
(72) Erfinder: Phaff, Rox, Dr., CH-4452 Itingen (CH)

(56) Entgegenhaltungen:
- EP-A- 0 242 169
- EP-A- 0 266 310
- CHEMICAL ABSTRACTS, vol. 98, no. 9, 28. Februar 1983, Columbus, Ohio, US; abstract no. 71864U, YU. M. CHUNAEV ET AL.: 'Interaction of a Fischer base with 8-hydroxy-1-naphthaldehydes. Carbon-13 NMR study of reaction products.' Seite 608;

## Beschreibung

Die vorliegende Erfindung betrifft Pyrrolin- bzw. indolinhaltige Phthalide, Verfahren zu ihrer Herstellung und ihre Verwendung als Farbbildner in druckempfindlichen oder wärmeempfindlichen Aufzeichnungsmaterialien.

Die erfindungsgemässen Phthalide entsprechen der allgemeinen Formel 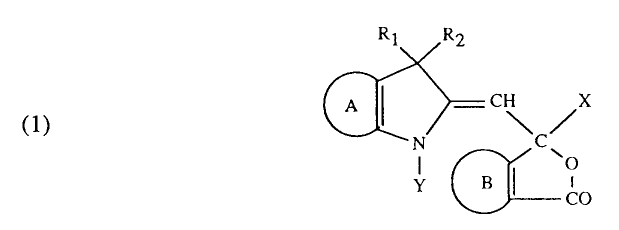
worin
R₁ und R₂, unabhängig voneinander, C₁-C₆-Alkyl, C₅-C₇-Cycloalkyl oder Benzyl oder zusammen C₄-C₆-Alkylen,
Y unsubstituiertes oder durch Halogen, Cyano, Hydroxy oder C₁-C₆-Alkoxy substituiertes Alkyl mit höchstens 12 Kohlenstoffatomen oder Benzyl,
X Wasserstoff, Alkyl mit 1 bis 12 Kohlenstoffatomen oder einen unsubstituierten oder durch Halogen, Cyano, C₁-C₆-Alkyl, C₅-C₆-Cycloalkyl, C₁-C₈-Acyl, -OR' oder -SR' substituierten Phenyl- oder Naphthylrest
R' unsubstituiertes oder durch Halogen, Hydroxy, Cyano oder C₁-C₆-Alkoxy substituiertes Alkyl mit höchstens 12 Kohlenstoffatomen, C₁-C₈-Acyl, C₅-C₆-Cycloalkyl, Phenyl oder Benzyl bedeuten; und der
Ring A einen gegebenenfalls durch Halogen, Cyano, C₁-C₆-Alkyl, C₁-C₆-Alkoxy oder C₁-C₆-Alkoxycarbonyl ein- oder mehrfach substituierten Benzol- oder Naphthalinring bedeutet und der Ring B einen unsubstituierten oder durch Halogen, Cyano, Nitro, C₁-C₆-Alkyl, C₁-C₆-Alkoxy, C₁-C₆-Alkylthio, C₁-C₆-Alkylcarbonyl, C₁-C₆-Alkoxycarbonyl, Amino, C₁-C₆-Alkylamino, Di-C₁-C₆-Alkylamino oder C₁-C₆-Alkylcarbonylamino substituierten Benzolring oder eine 6-gliedrigen stickstoffhaltigen heterocyclischen Ring mit aromatischem Charakter oder einen Naphthalin-, Chinolin- oder Chinoxalinring bedeutet, mit der Massgabe, dass die Verbindung 3-[(1,3-Dihydro-1,3,3-trimethyl-2H-indol-2-yliden)methyl]-1(3H)-isobenzofuranon nicht mit umfaßt ist, und dass C₁-C₈-Acyl Formyl, C₁-C₆-Alkylcarbonyl, C₁-C₆-Alkylsulfonyl, gegebenenfalls durch Halogen, Methyl, Methoxy oder Ethoxy substituiertes Benzoyl oder Phenylsulfonyl bedeutet.

Der Ring A stellt vorteilhafterweise einen unsubstituierten oder durch Halogen, Cyano oder C₁-C₆-Alkyl substituierten Benzolkern dar. Besonders bevorzugt ist ein unsubstituierter oder durch Halogen substituierter Benzolkern.

Die durch B wiedergegebenen bevorzugten 6-gliedrigen aromatischen oder heterocyclischen Reste sind der 2,3-Pyridino-, 3,4-Pyridino-, 2,3-Pyrazino-, 2,3-Chinoxalino-, 1,2-Naphthalino-, 2,3-Naphthalino- oder 1,2-Benzorest, der unsubstituiert oder durch Halogen, wie Chlor oder Brom, Nitro, C₁-C₆-Alkyl, C₁-C₆-Alkoxy, C₁-C₆-Alkylthio oder eine wie vorstehend definiert gegebenenfalls substituierte Aminogruppe substituiert ist, wobei der unsubstituierte oder durch Chloratome, C₁-C₆-Alkoxy oder Di-C₁-C₆-Alkylamino, besonders Dimethylamino substituierte 1,2-Benzorest besonders bevorzugt ist.

Die Reste R₁ und R₂ können voneinander verschieden sein oder sind vorzugsweise identisch. R₁ und R₂ bedeuten vorzugsweise C₁-C₆-Alkyl und vor allem beide Methyl. Bedeuten R₁ und R₂ zusammen Alkylen, so weisen sie mit Vorteil 4 oder 5 Kohlenstoffatome auf und können mit dem sie verbindenden Kohlenstoffatom einen Cyclopentan-, Cycloheptan- oder vorzugsweise einen Cyclohexanring bilden.

Beispiele für Cycloalkyl in der Bedeutung von R₁ und R₂ sind Cycloheptyl, Cyclopentyl oder vorzugsweise Cyclohexyl.

Stellt der N-Substituent Y Alkylreste dar, so können diese geradkettig oder verzweigt sein. Beispiele hierfür sind Methyl, Ethyl, n-Propyl, Isopropyl, n-Butyl, 1-Methylbutyl, t-Butyl, sek.Butyl, Amyl, Isopentyl, n-Hexyl, 2-Ethylhexyl, Isooctyl, n-Octyl, 1,1,3,3-Tetramethylbutyl, Nonyl, Isononyl, 3-Ethylheptyl, Decyl oder n-Dodecyl.

Ist der Alkylrest in Y substituiert, so handelt es sich vor allem um Cyanoalkyl, Halogenalkyl, Hydroxyalkyl, Alkoxyalkyl jeweils vorzugsweise mit insgesamt 2 bis 8 Kohlenstoffatomen, wie z.B. 2-Cyanoethyl, 2-Chlorethyl, 2-Hydroxyethyl, 2-Methoxyethyl, 2-Ethoxyethyl, 2,3-Dihydroxypropyl, 2-Hydroxy-3-chlorpropyl, 3-Methoxypropyl, 4-Methoxybutyl oder 4-Propoxybutyl.

Der N-Substituent Y ist vorzugsweise C₁-C₆-Alkyl und vor allem Methyl.

Als Alkyl stellt X beispielsweise die für Alkylreste oben aufgezählten Substituenten dar.

Als Arylrest stellt X vorzugsweise einen substituierten Phenylrest der Formel

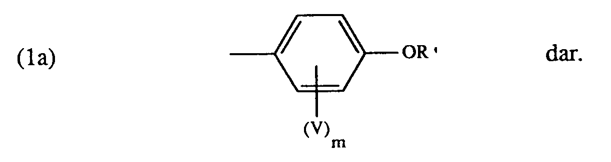

Hierbei bedeutet R' unsubstituiertes oder durch Halogen, Hydroxy, Cyano oder C₁-C₆-Alkoxy substituiertes Alkyl mit höchstens 12 Kohlenstoffat C₁-C₈-Acyl, C₅-C₆-Cycloalkyl, Phenyl oder Benzyl. V bedeutet Wasserstoff, Halogen, C₁-C₆-Alkyl, C₁-C₁₂-Alkoxy, C₁-C₈-Acyloxy, Benzyloxy oder Phenyloxy. m ist 1 oder 2. Ein V befindet sich vorzugsweise in Ortho-Stellung zur Verknüpfungsstelle.

Der Acyloxyrest in V ist beispielsweise Formyloxy, C₁-C₆-Alkylcarbonyloxy, wie z.B. Acetyloxy oder Propionyloxy, oder Benzoyloxy. Als C₁-C₁₂-Alkoxyrest kann V eine geradkettige oder verzweigte Gruppe sein, wie z.B. Methoxy, Ethoxy, Isopropoxy, n-Butoxy, tert-Butoxy, Amyloxy, 1,1,3,3-Tetramethylbutoxy, n-Hexyloxy, n-Octyloxy oder Dodecyloxy.

Der Substituent X ist vorzugsweise Wasserstoff.

C₁-C₆-Alkyl, C₁-C₆-Alkoxy und C₁-C₆-Alkylthio stellen solche Gruppen oder Gruppenbestandteile dar, die 1 bis 6, insbesondere 1 bis 4 Kohlenstoffatome aufweisen. Beispiele für C₁-C₆-Alkyl sind Methyl, Ethyl, n-Propyl, Isopropyl, n-Butyl, sek.Butyl, Amyl, Isoamyl oder Hexyl bzw. Methoxy, Ethoxy, Isopropoxy, Isobutoxy, tert.Butoxy oder Amyloxy bzw. Methylthio, Ethylthio, Propylthio oder Butylthio.

Halogen bedeutet beispielsweise Fluor, Brom oder vorzugsweise Chlor.

C₁-C₆-Alkylcarbonyl sind z.B. Acetyl oder Propionyl. C₁-C₆-Alkylsulfonyl sind z.B. Methylsulfonyl oder Ethylsulfonyl. Benzoyl und Phenylsulfonyl können durch Halogen, Methyl, Methoxy oder Ethoxy substituiert sein.

Besonders wichtige indolinhaltige Phthalide entsprechen der Formel 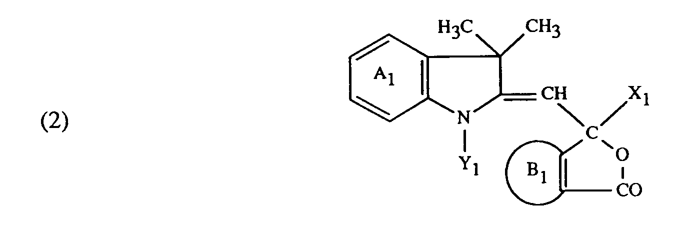
worin
der Ring A₁ unsubstituiert oder durch Halogen, Cyano, C₁-C₆-Alkyl oder C₁-C₆-Alkoxy substituiert ist und der Ring B₁ einen gegebenenfalls durch Halogen, Cyano, C₁-C₆-Alkyl, C₁-C₆-Alkoxy, C₁-C₆-Alkylcarbonylamino oder Di-C₁-C₆-Alkylamino substituierten Benzol- oder Pyridinring,
Y₁ C₁-C₆-Alkyl, Benzyl oder durch Halogen, Hydroxy, Cyano, C₁-C₆-Alkoxy substituiertes C₁-C₆-Alkyl und
X₁ Wasserstoff oder einen substituierten Phenylrest der Formel 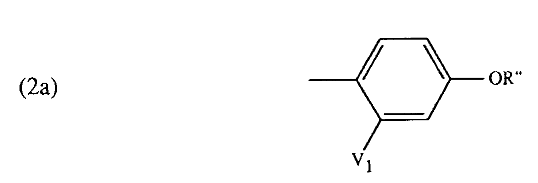
wobei R'' C₁-C₆-Alkyl oder Phenyl und V₁ Wasserstoff, Halogen oder C₁-C₆-Alkoxy darstellen, bedeuten, mit der Massgabe, dass die Verbindung 3-[(1,3-Dihydro-1,3,3-trimethyl-2H-indol-2-yliden)methyl]-1(3H)-isobenzofuranon nicht mit umfaßt ist.

Unter den Phthalidverbindungen der Formel (2) sind diejenigen, in denen X₁ Wasserstoff oder einen Phenylrest der Formel (2a), in der R'' C₁-C₆-Alkyl und V₁ Wasserstoff darstellen, und der Ring B₁ einen gegebenenfalls durch C₁-C₆-Alkoxy substituierten Benzolring bedeuten, bevorzugt. X₁ ist in Formel (2) insbesondere Wasserstoff.

Von besonderem Interesse sind indolinhaltige Phthalidverbindungen der Formel 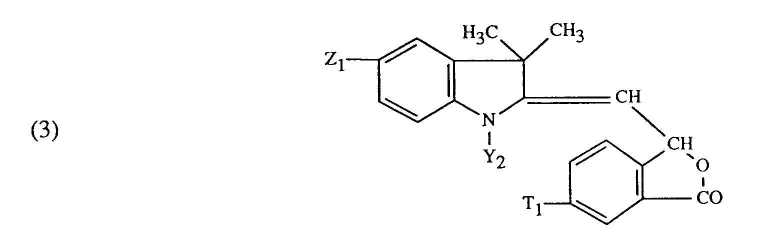
worin
T₁ Wasserstoff oder C₁-C₆-Alkoxy, vor allem Methoxy,
Y₂ C₁-C₆-Alkyl, in erster Linie Methyl, und
Z₁ Wasserstoff oder Halogen, wie Chlor, bedeuten, mit der Massgabe, dass die Verbindung 3-[(1,3-Dihydro-1,3,3-trimethyl-2H-indol-2-yliden)methyl]-1(3H)-isobenzofuranon nicht mit umfaßt ist.

Erfindungsgemässe Verbindungen der Formel (1) können hergestellt werden, indem man eine 2-Methylenpyrrolinverbindung der Formel 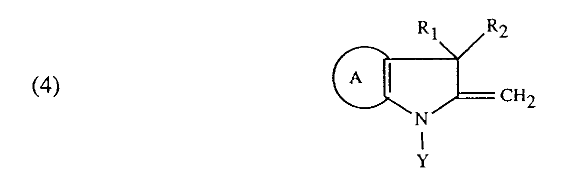
worin A, R₁, R₂ und Y die angegebene Bedeutung haben, mit einer Aldehydsäure bzw. Ketosäure der Formel 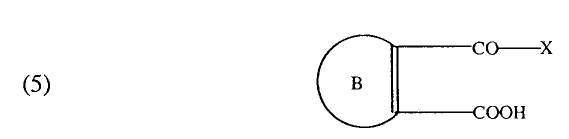
worin X und B die angegebene Bedeutung haben, umsetzt.

Die Umsetzung wird zweckmässigerweise in den bei der Reaktionstemperatur flüssigen organischen Lösungsmitteln z.B. Acetonitril oder Propionitril, Benzol, Xylol, Toluol, Chlorbenzol oder Nitrobenzol durchgeführt. Vorzugsweise erfolgt die Kondensation bei Temperaturen im Bereich von 20 bis 120°C, Vorzugsweise 40 bis 80°C.

Die für die Herstellung der erfindungsgemässen Phthalide benötigten Ausgangsprodukte der Formeln (4) und (5) sind grösstenteils bekannt.

Geeignete Ausgangsprodukte der Formel (4) sind:
1,3,3-Trimethyl-2-methylenindolin,
1,3,3-Trimethyl-5-chlor-2-methylenindolin,
1,3,3-Trimethyl-2-methylen-benzoindolin,
1,3,3,4,5-Pentamethyl-2-methylenindolin.

Zu spezifischen Beispielen der Ausgangsprodukte der Formel (5) gehören:
Phthaläldehydsäure
5-Methoxyphthalaldehydsäure,
5-Dimethylaminophthalaldehydsäure
4'-Methoxy-4-dimethylamino-benzophenon-2-carbonsäure,
4,4'-Bis-methoxy-benzophenon-2-carbonsäure,
4-Methoxy-benzophenon-2-carbonsäure,
4-Dimethylamino-benzophenon-2-carbonsäure,
4-Diethylamino-benzophenon-2-carbonsäure.

Die pyrrolinhaltigen Phthalide der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach der Bedeutung von X und dem verwendeten Entwickler intensive gelbe, orange oder rote Farbtöne, die sublimations- und lichtecht sind. Die Phthalide der Formeln (1) bis (3) sind auch sehr wertvoll im Gemisch mit einem oder mehreren anderen bekannten Farbbildnern, z.B. 3,3-(Bis-aminophenyl-phthaliden, wie CVL, 3-Indolyl-3-aminophenylaza- oder -diazaphthaliden, (3,3-Bis-indolyl-)-phthaliden, 3-Aminofluoranen, 2,6-Diaminofluoranen, 2,6-Diamino-3-methyl-fluoranen, 3,6-Bisalkoxyfluoranen, 3,6-Bisdiarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um blaue, marine-blaue, graue oder schwarze Färbungen zu ergeben.

Die Phthalide der Formeln (1) bis (3) zeigen sowohl auf aktivierten Tonen, wie auch auf phenolischen Unterlagen eine ausgezeichnete Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Nach Belichtung in CB-Blatt weisen sie eine geringe Abnahme der Farbstarke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele Für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminium- phosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsbustituierte Phenole, Resorcine, Salicylsäuren, wie z.B. 3,5-Bis-(α,α-dimethylbenzyl-)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt, Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester, wie z.B. Tricresylphosphat, Di-n-butylphthalat, Dioctylphthalat, Trichlorethylphosphat, aromatische Ether, wie Benzylphenylether, Kohlenwasserstofföle, wie Paraffin oder Kerosin, aromatische Kohlenwasserstoffe, wie z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-C₁-C₃-alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Phthalide dadurch aus, dass sie löslich sind und pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor (Farbentwickler) in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Vorzugsweise besteht das Durchschreibematerial auch darin, dass es eine kapselfreie, den Farbbildner enthaltende Schicht und eine farbentwickelnde Schicht, die als Farbentwickler mindestens ein anorganisches Metallsalz eines mehrwertigen Metalles, vor allem Halogenide oder Nitrate, wie z.B. Zinkchlorid, Zinnchlorid, Zinknitrat oder deren Gemische enthält, aufweist.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen oder mehrere Farbbildner, und einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-BS 1 251 348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenyl-sulfon, 4'-Hydroxy-4-methyldiphenylsulfon, 4'-Hydroxy-4-isopropoxydi-phenylsulfon, 4,4'-Cyclo-hexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Phthalide und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titzndioxyd, Zinkoxyd, Aluminiumhydroxyd, Calciumcarbonat (z.B. Kreide), Magnesiumcarbonat, Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Stearinsäureamid, Bis-stearoylethylendiamid, Phthalsäureanhydrid, Metallstearate, wie z.B. Zinkstearat, Phthalsäurenitril, Dimethylterephthalat, Dibenzylterephthalat, Benzyldiphenyl oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht.
Beispiel 1: 1,8 g 5-Methoxyphthalaldehydsäure und 1,93 g 1,3,3-Trimethyl-2-methylenindolin (Fischerbase) werden in 15 ml Toluol 1 Stunde bei 50°C gerührt. Das Reaktionsgemisch wird eingeengt und an Kieselgel chromatographiert Man erhält 3,24 g einer Verbindung der Formel 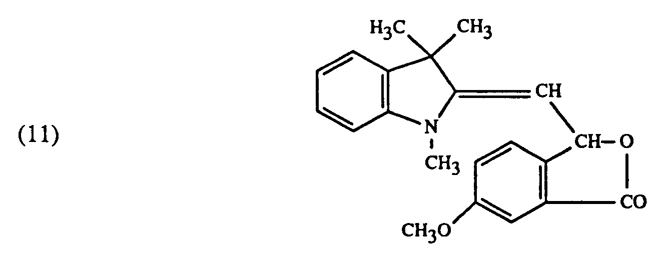 mit einem Schmelzpunkt von 75-85°C. Diese Verbindung erzeugt auf einem mit Aktivton beschichteten Papier eine lichtechte gelbe Farbe (λₘₐₓ = 440 nm).
Beispiel 2: 1,53 g Phthalaldehydsäure und 2,23 g 1,3,3-Trimethyl-2-methylen-5-chlorindolin werden in 20 ml Toluol 30 Minuten bei 50°C gerührt. Das Reaktionsgemisch wird abgekühlt, wobei das Produkt auskristallisiert Dieses wird abfiltriert und getrocknet. Man erhält 1,0 g einer Verbindung der Formel 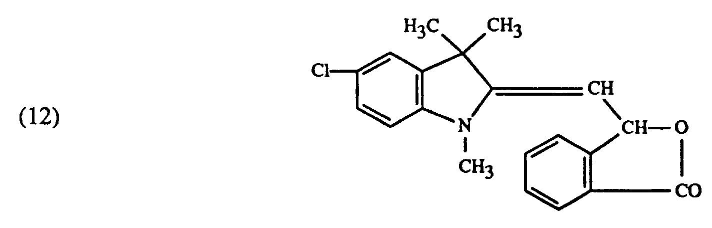 mit einem Schmelzpunkt von 191- 192°C. Auf einem mit Aktivton beschichteten Papier ergibt diese Verbindung eine gelbe Farbe.
Beispiel 3: Herstellung eines druckempfindlichen Kopierpapiers.
   Eine Lösung von 3 g des Phthalides der Formel (11) (Beispiel 1) in 80 g Diisopropylnaphthalin und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive gelbe Kopie, die ausgezeichnet lichtecht ist.
Beispiel 4: 1 g des Phthalides der Formel (12) gemäss Beispiel 2 wird in 17 g Toluol gelöst. Zu dieser Lösung gibt man unter Rühren 12 g Polyvinylacetat, 8 g Calciumcarbonat und 2 g Titandioxid Die erhaltene Suspension wird im Gewichtsverhältnis 1:1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich sofort auf dem mit dem Farbbildner beschichteten Blatt eine intensive und lichtechte gelbe Farbe.
Beispiel 5: Herstellung eines wärmeempfindlichen Aufzeichnungsmaterials
   In einer Kugelmühle werden 32 g 4,4'-Isopropylidendiphenol (Bisphenol A), 3,8 g Distearylamid des Ethylendiamins, 39 g Kaolin, 20g eines zu 88 % hydrolysierten Polyvinylalkohols und 500 ml Wasser gemahlen bis die Teilchengrösse ca. 5 µm beträgt. In einer zweiten Kugelmühle werden 6 g des Phthalides der Formel (11) gemäss Beispiel 1, 3 g eines zu 88 % hydrolysierten Polyvinylalkohols und 60 ml Wasser zu einer Teilchengrösse von ca. 3 µm gemahlen.
   Die beiden Dispersionen werden zusammengegeben und mit einem Trockenauftragsgewicht von 5,5 g/m² auf ein Papier gestrichen. Durch Berührung des Papiers mit einem erhitzten Kugelschreiber wird eine intensive gelbe Farbe erhalten, die eine ausgezeichnete Licht- und Sublimierechtheit hat.
Beispiel 6: 1,1 g des Farbbildners gemäss EP-A-90810714.7 der Formel 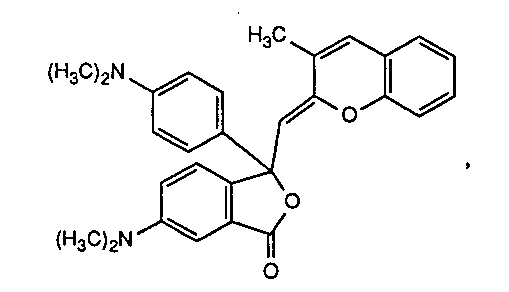 2,3 g 3,3-Bis(1'-n-octyl-2'-methylindol-3'-yl)phthalid und 0,6 g des Farbbildners gemäss Beispiel 1 werden in 100 g partiell hydriertem Terphenyl bei 70-80°C gelöst. Die abgekühlte Lösung wird mit einem Tiefdruckgerät auf ein vorgeleimtes und mit aktiviertem Clay beschichtetes Papier gebracht. Es entwickelt sich sofort eine intensive und lichtechte Schwarzfärbung.

## Patentansprüche

1. Phthalidverbindungen der Formel 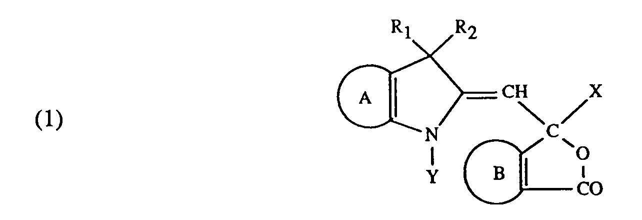 worin
R₁ und R₂, unabhängig voneinander, C₁-C₆-Alkyl, C₅-C₇-Cycloalkyl oder Benzyl oder zusammen C₄-C₆-Alkylen,
Y unsubstituiertes oder durch Halogen, Cyano, Hydroxy oder C₁-C₆-Alkoxy substituiertes Alkyl mit höchstens 12 Kohlenstoffatomen oder Benzyl,
X Wasserstoff, Alkyl mit 1 bis 12 Kohlenstoffatomen oder einen unsubstituierten oder durch Halogen, Cyano, C₁-C₆-Alkyl, C₅-C₆-Cycloalkyl, C₁-C₈-Acyl, -OR' oder -SR' substituierten Phenyl- oder Naphthylrest,
R' unsubstituiertes oder durch Halogen, Hydroxy, Cyano oder C₁-C₆-Alkoxy substituiertes Alkyl mit höchstens 12 Kohlenstoffatomen, C₁-C₈-Acyl, C₅-C₆-Cycloalkyl, Phenyl oder Benzyl bedeuten; und der
Ring A einen gegebenenfalls durch Halogen, Cyano, C₁-C₆-Alkyl, C₁-C₆-Alkoxy oder C₁-C₆-Alkoxycarbonyl ein- oder mehrfach substituierten Benzol- oder Naphthalinring bedeutet und der Ring B einen unsubstituierten oder durch Halogen, Cyano, Nitro, C₁-C₆-Alkyl, C₁-C₆-Alkoxy, C₁-C₆-Alkylthio, C₁-C₆-Alkylcarbonyl, C₁-C₆-Alkoxycarbonyl, Amino, C₁-C₆-Alkylamino, Di-C₁-C₆-Alkylamino oder C₁-C₆-Alkylcarbonylamino substituierten Benzolring oder einen 6-gliedrigen stickstoffhaltigen heterocyclischen Ring mit aromatischem Charakter oder einen Naphthalin-, Chinolin- oder Chinoxalinring bedeutet, mit der Massgabe, dass die Verbindung 3-[(1,3-Dihydro-1,3,3-trimethyl-2H-indol-2-yliden)methyl]-1(3H)-iso benzofuranon nicht mit umfaßt ist, und dass C₁-C₈-Acyl Formyl, C₁-C₆-Alkylcarbonyl, C₁-C₆-Alkylsulfonyl, gegebenenfalls durch Halogen, Methyl, Methoxy oder Ethoxy substituiertes Benzoyl oder Phenylsulfonyl bedeutet.

2. Phthalidverbindungen gemäss Anspruch 1 dadurch gekennzeichnet, dass in Formel (1) Y C₁-C₆-Alkyl bedeutet.

3. Phthalidverbindungen gemäss einem der Ansprüche 1 und 2, dadurch gekennzeichnet, dass in Formel (1) X Wasserstoff bedeutet.

4. Phthalidverbindungen gemäss einem der Ansprüche 1 bis 3, dadurch gekennzeichnet, dass in Formel (1) der Ring A einen unsubstituierten oder durch Halogen oder C₁-C₆-Alkyl substituierten Benzolkern darstellt.

5. Phthalidverbindungen gemäss einem der Ansprüche 1 bis 4 dadurch gekennzeichnet, dass in Formel (1) der Ring B einen gegebenenfalls substituierten Benzol-, Naphthalin-, Pyridin-, Pyrazin-, Chinoxalin- oder Chinolinring darstellt.

6. Phthalidverbindungen gemäss einem der Ansprüche 1 bis 5 dadurch gekennzeichnet, dass in Formel (1) der Ring B einen unsubstituierten oder durch Chlor, C₁-C₆-Alkoxy oder Di-C₁-C₆-Alkylamino substituierten Benzolring darstellt.

7. Phthalidverbindungen gemäss Anspruch 1, dadurch gekennzeichnet, dass sie der Formel 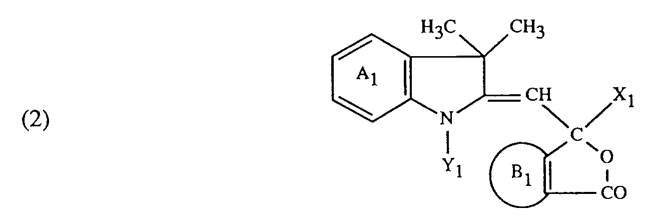 entsprechen, worin
der Ring A₁ unsubstituiert oder durch Halogen, Cyano, C₁-C₆-Alkyl oder C₁-C₆-Alkoxy substituiert ist und der Ring B₁ einen gegebenenfalls durch Halogen, Cyano, C₁-C₆-Alkyl, C₁-C₆-Alkoxy, C₁-C₆-Alkylcarbonylamino oder Di-C₁-C₆-Alkylamino substituierten Benzol- oder Pyridinring,
Y₁ C₁-C₆-Alkyl, Benzyl oder durch Halogen, Hydroxy, Cyano, C₁-C₆-Alkoxy substituiertes C₁-C₆-Alkyl und
X₁ Wasserstoff oder einen substituierten Phenylrest der Formel 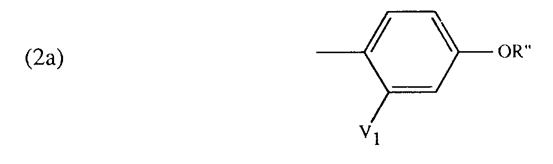 wobei R'' C₁-C₆-Alkyl oder Phenyl und V₁ Wasserstoff, Halogen oder C₁-C₆-Alkoxy darstellen, bedeuten.

8. Phthalidverbindungen gemäss Anspruch 7, dadurch gekennzeichnet, dass in Formel (2) X₁ Wasserstoff oder einen Phenylrest der Formel (2a), in der R'' C₁-C₆-Alkyl und V₁ Wasserstoff darstellen, und der Ring B₁ einen gegebenenfalls durch C₁-C₆-Alkoxy substituierten Benzolring bedeuten.

9. Phthalidverbindungen gemäss Anspruch 1 dadurch gekennzeichnet dass sie der Formel 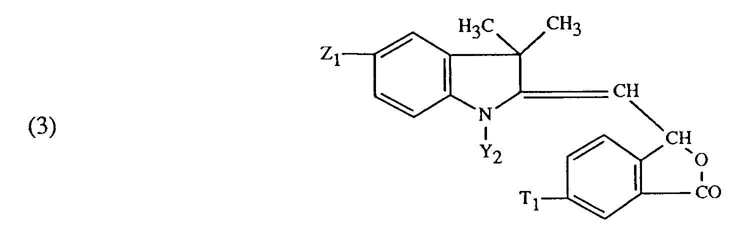 entsprechen, worin
T₁ Wasserstoff oder C₁-C₆-Alkoxy,
Y₂ C₁-C₆-Alkyl und
Z₁ Wasserstoff oder Halogen bedeuten.

10. Verfahren zur Herstellung von Phthalidverbindungen gemäss Anspruch 1, dadurch gekennzeichnet, dass man eine 2-Methylenpyrrolinverbindung der Formel 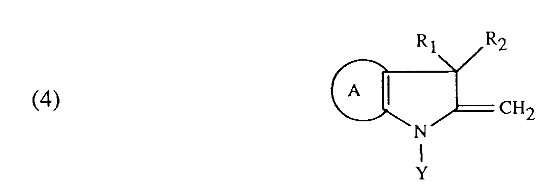 worin A, R₁, R₂ und Y die in Anspruch 1 angegebene Bedeutung haben, mit einer Aldehydsäure bzw. Ketosäure der Formel 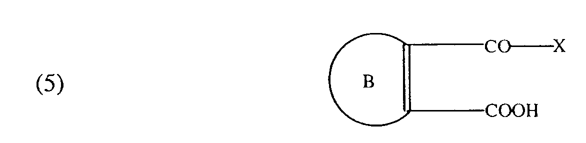 worin X und B die in Anspruch 1 angegebene Bedeutung haben, umsetzt.

11. Druck- oder wärmeempfindliches Aufzeichnungsmaterial, dadurch gekennzeichnet, dass es in seinem Farbreaktantensystem als Farbbildner mindestens eine Phthalidverbindung der Formel (1) gemäss Anspruch 1, die Verbindung 3-[(1,3-Dihydro-1,3,3-trimethyl-2H-indol-2-yliden)methyl]-1(3H)-isobenzofuranon mit einbegriffen, enthält.

12. Druckempfindliches Aufzeichnungsmaterial gemäss Anspruch 11, dadurch gekennzeichnet, dass es die Phthalidverbindung, gelöst in einem organischen Lösungsmittel, und mindestens einen festen Elektronenakzeptor enthält.

13. Druckempfindliches Aufzeichnungsmaterial gemäss einem der Ansprüche 11 und 12, dadurch gekennzeichnet, dass die Phthalidverbindung in Mikrokapseln eingekapselt ist.

14. Druckempfindliches Aufzeichnungsmaterial gemäss Anspruch 13, dadurch gekennzeichnet, dass die eingekapselte Phthalidverbindung in Form einer Schicht auf der Rückseite eines Übertragungsblattes und der Elektronenakzeptor in Form einer Schicht auf der Vorderseite des Empfangsblattes vorhanden sind.

15. Warmeempfindliches Aufzeichnungsmaterial gemäss Anspruch 11, dadurch gekennzeichnet, dass es in mindestens einer Schicht mindestens eine Phthalidverbindung gemäss Anspruch 11, einen Elektronenakzeptor und gegebenenfalls ein Bindemittel und/oder Wachs enthält.

16. Aufzeichnungsmaterial gemäss einem der Ansprüche 11 bis 15, dadurch gekennzeichnet, dass die Phthalidverbindung gemeinsam mit einem oder mehreren anderen Farbbildnern enthalten ist.

## Claims

1. A phthalide compound of formula 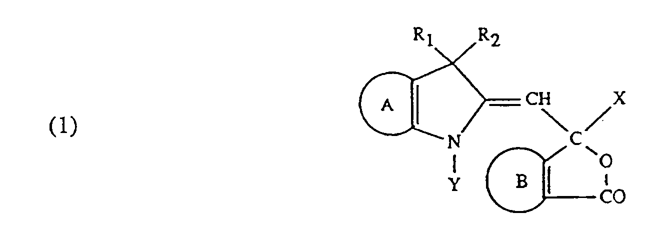 wherein
R₁ and R₂ are each independently of the other C₁-C₆alkyl, C₅-C₇cycloalkyl or benzyl or together are C₄-C₆alkylene,
Y is alkyl of not more than 12 carbon atoms, unsubstituted or substituted by halogen, cyano, hydroxy or C₁-C₆alkoxy, or is benzyl, X is hydrogen, alkyl of 1 to 12 carbon atoms or a phenyl or naphthyl radical which is unsubstituted or substituted by halogen, cyano, C₁-C₆alkyl, C₅-C₆cycloalkyl, C₁-C₈acyl, -OR' or -SR',
R' is alkyl of not more than 12 carbon atoms which is unsubstituted or substituted by halogen, hydroxy, cyano or C₁-C₆alkoxy, or is C₅-C₆cycloalkyl, phenyl or benzyl; and the
ring A is a benzene or naphthalene ring which is unsubstituted or mono- or polysubstituted by halogen, cyano C₁-C₆alkyl, C₁-C₆alkoxy or C₁-C₆alkoxycarbonyl, and the ring B is a benzene ring which is unsubstituted or substituted by halogen, cyano, nitro, C₁-C₆alkyl, C₁-C₆alkoxy, C₁-C₆alkylthio, C₁-C₆alkylcarbonyl, C₁-C₆alkoxycarbonyl, amino, C₁-C₆alkylamino, di-C₁-C₆alkylamino or C₁-C₆alkylcarbonylamino, or is a 6-membered nitrogen-containing heterocyclic ring of aromatic character, or is a naphthalene, quinoline or quinoxaline ring, with the proviso that the compound 3-[(1,3-dihydro-1,3,3-trimethyl-2H-indol-2-ylidene)methyl]-1(3H)-isobenzofuranone is not included, and that C₁-C₈acyl is formyl, C₁-C₆alkylcarbonyl, C₁-C₆alkylsulfonyl, unsubstituted or halogen-, methyl-, methoxy- or ethoxy-substituted benzoyl or phenylsulfonyl.

2. A phthalide compound according to claim 1, wherein Y in formula (1) is C₁-C₆alkyl.

3. A phthalide compound according to either of claims 1 and 2, wherein X in formula (1) is hydrogen.

4. A phthalide compound according to one of claims 1 to 3, wherein the ring A in formula (1) is a benzene ring which is unsubstituted or substituted by halogen or C₁-C₆alkyl.

5. A phthalide compound according to one of claims 1 to 4, wherein the ring B in formula (1) is an unsubstituted or substituted benzene, naphthalene, pyridine, pyrazine, quinoxaline or quinoline ring.

6. A phthalide compound according to one of claims 1 to 5, wherein the ring B in formula (1) is a benzene ring which is unsubstituted or substituted by chloro, C₁-C₆alkoxy or di-C₁-C₆alkylamino.

7. A phthalide compound according to claim 1, which is of formula 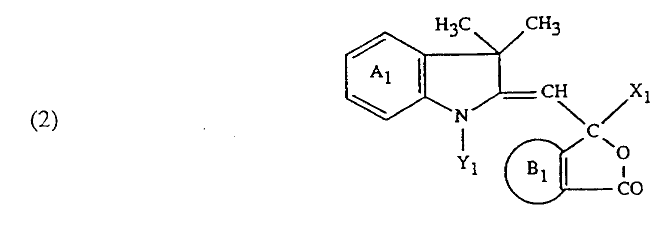 wherein
the ring A₁ is unsubstituted or substituted by halogen, cyano, C₁-C₆alkyl or C₁-C₆alkoxy, and the ring B₁ is a benzene or pyridine ring which is unsubstituted or substituted by halogen, cyano, C₁-C₆alkyl, C₁-C₆alkoxy, C₁-C₆alkylcarbonylamino or di-C₁-C₆alkylamino,
Y₁ is C₁-C₆alkyl, benzyl or C₁-C₆alkyl which is substituted by halogen, hydroxy, cyano, C₁-C₆alkoxy, and
X₁ is hydrogen or a substituted phenyl radical of formula 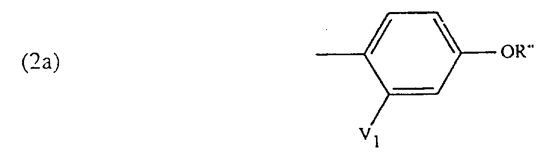 where R'' is C₁-C₆alkyl or phenyl and V₁ is hydrogen, halogen or C₁-C₆alkoxy.

8. A phthalide compound according to claim 7, wherein X₁ in formula (2) is hydrogen or a phenyl radical of formula (2a) in which R'' is C₁-C₆alkyl and V₁ is hydrogen, and the ring B₁ is an unsubstituted or C₁-C₆alkoxy-substituted benzene ring.

9. A phthalide compound according to claim 1, which is of formula 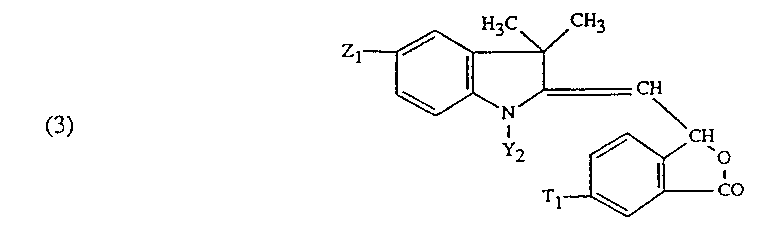 wherein
T₁ is hydrogen or C₁-C₆alkoxy,
Y₂ is C₁-C₆alkyl, and
Z₁ is hydrogen or halogen.

10. A process for the preparation of a phthalide compound according to claim 1, which comprises reacting a 2-methylenepyrroline compound of formula 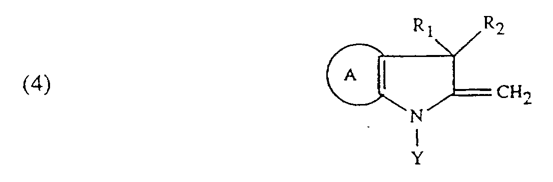 wherein A, R₁, R₂ and Y have the meaning given in claim 1 with an aldehyde acid or keto acid of formula 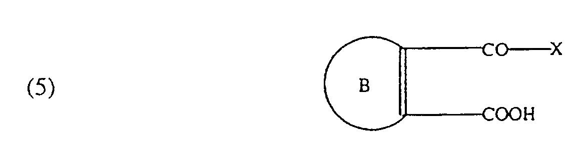 wherein X and B have the meaning given in claim 1.

11. A pressure-sensitive or heat-sensitive recording material which comprises in its colour reactant system as colour former, at least one phthalide compound of formula (1) according to claim 1, including 3-[(1,3-dihydro-1,3,3-trimethyl-2H-indol-2-ylidene)methyl]-1(3H)-isobenzofuranone.

12. A pressure-sensitive recording material according to claim 11, which comprises the phthalide compound dissolved in an organic solvent, and at least one solid electron acceptor.

13. A pressure-sensitive recording material according to either of claims 11 and 12, wherein the phthalide compound is encapsulated in microcapsules.

14. A pressure-sensitive recording material according to claim 13, wherein the encapsulated phthalide compound is present in the form of a layer on the back of a transfer sheet and the electron acceptor is present in the form of a layer on the face of the receiving sheet.

15. A heat-sensitive recording material according to claim 11, which comprises in at least one layer at least one phthalide compound according to claim 11, one electron acceptor and if desired a binder and/or wax.

16. A recording material according to one of claims 11 to 15, which comprises the phthalide compound together with one or more other colour formers.

## Revendications

1. Composés de type phtalide, de formule 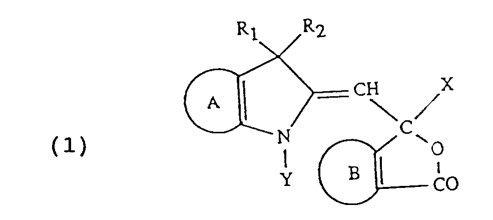 dans laquelle
R₁ et R₂ représentent chacun, indépendamment l'un de l'autre, un groupe alkyle en C₁₋₆, cycloalkyle en C₅₋₇ ou benzyle ou représentent conjointement un groupe alkylène en C₄₋₆,
Y représente un groupe alkyle comportant au plus 12 atomes de carbone, portant ou non des substituants halogéno, cyano, hydroxy ou alcoxy en C₁₋₆, ou un groupe benzyle,
X représente un atome d'hydrogène ou un groupe alkyle comportant de 1 à 12 atomes de carbone, ou encore un groupe phényle ou naphtyle portant ou non des substituants halogéno, cyano, alkyle en C₁₋₆, cycloalkyle en C₅₋₆, acyle en C₁₋₈, -OR' ou -SR',
où R' représente un groupe alkyle comportant au plus 12 atomes de carbone, portant ou non des substituants halogéno, hydroxy, cyano ou alcoxy en C₁₋₆, ou un groupe acyle en C₁₋₈, cycloalkyle en C₅₋₆, phényle ou benzyle,
le cycle A est un cycle benzénique ou naphtalénique, portant éventuellement un ou plusieurs substituants halogéno, cyano, alkyle en C₁₋₆, alcoxy en C₁₋₆ ou (alcoxy en C₁₋₆)-carbonyle,
et le cycle B est un cycle benzénique portant ou non des substituants halogéno, cyano, nitro, alkyle en C₁₋₆, alcoxy en C₁₋₆, alkylthio en C₁₋₆, (alkyle en C₁₋₆)-carbonyle, (alcoxy en C₁₋₆)-carbonyle, amino, (alkyle en C₁₋₆)-amino, di-(alkyle en C₁₋₆)-amino ou (alkyle en C₁₋₆)-carbonylamino, ou un hétérocycle azoté à 6 chaînons et à caractère aromatique, ou encore un cycle de naphtalène, de quinoline ou de quinoxaline,
sous réserve que soit exclu le composé 3-[(1,3-dihydro1,3,3-triméthyl-2H-indole-2-ylidène)méthyl]-1(3H)-isobenzofuranone,
et que le groupe acyle en C₁₋₈ représente un groupe formyle, (alkyle en C₁₋₆)-carbonyle, (alkyle en C₁₋₆)-sulfonyle, ou phénylsulfonyle ou benzoyle portant éventuellement des substituants halogéno, méthyle, méthoxy ou éthoxy.

2. Composés de type phtalide, conformes à la revendication 1**, caractérisés** en ce que dans la formule (1), Y représente un groupe alkyle en C₁₋₆.

3. Composés de type phtalide, conformes à l'une des revendications 1 et 2, **caractérisés** en ce que dans la formule (1), X représente un atome d'hydrogène.

4. Composés de type phtalide, conformes à l'une des revendications 1 à 3, **caractérisés** en ce que dans la formule (1), le cycle A est un cycle benzénique portant ou non des substituants halogéno ou alkyle en C₁₋₆.

5. Composés de type phtalide, conformes à l'une des revendications 1 à 4, **caractérisés** en ce que dans la formule (1), le cycle B est un cycle de type benzène, naphtalène, pyridine, pyrazine, quinoxaline ou quinoline, portant éventuellement des substituants.

6. Composés de type phtalide, conformes à l'une des revendications 1 à 5, **caractérisés** en ce que dans la formule (1), le cycle B est un cycle benzénique portant ou non des substituants chloro, alcoxy en C₁₋₆ ou di-(alkyle en C₁₋₆)-amino.

7. Composés de type phtalide, conformes à la revendication 1, **caractérisés** en ce qu'ils correspondent à la formule 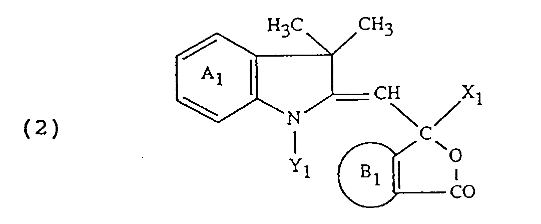 dans laquelle
le cycle A₁ porte des substituants halogéno, cyano, alkyle en C₁₋₆ ou alcoxy en C₁₋₆ ou n'en porte pas,
le cycle B₁ est un cycle de benzène ou de pyridine portant éventuellement des substituants halogéno, cyano, alkyle en C₁₋₆, alcoxy en C₁₋₆, (alkyle en C₁₋₆)-carbonylamino ou di(alkyle en C₁₋₆)-amino,
Y₁ représente un groupe alkyle en C₁₋₆ ou benzyle, ou encore un groupe alkyle en C₁₋₆ portant des substituants halogéno, hydroxy, cyano ou alcoxy en C₁₋₆,
et X₁ représente un atome d'hydrogène ou un groupe phényle substitué, de formule 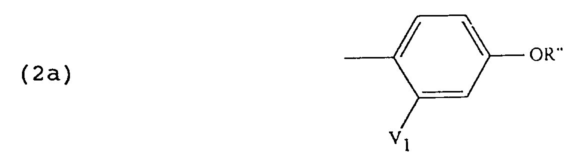 dans laquelle R'' représente un groupe alkyle en C₁₋₆ ou phényle et V₁ représente un atome d'hydrogène ou d'halogène ou un groupe alcoxy en C₁₋₆.

8. Composés de type phtalide, conformes à la revendication 7, **caractérisés** en ce que dans la formule (2), X₁ représente un atome d'hydrogène ou un groupe phényle de formule (2a) où R'' représente un groupe alkyle en C₁₋₆ et V₁ représente un atome d'hydrogène, et le cycle B₁ est un cycle benzénique portant éventuellement des substituants alcoxy en C₁₋₆.

9. Composés de type phtalide, conformes à la revendication 1, **caractérisés** en ce qu'ils correspondent à la formule 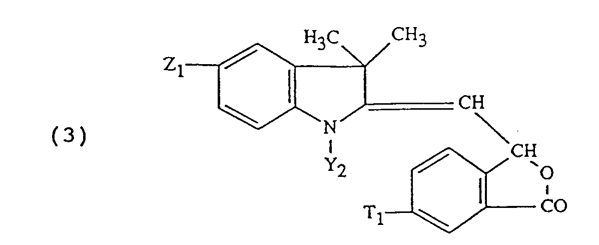 dans laquelle T₁ représente un atome d'hydrogène ou un groupe alcoxy en C₁₋₆, Y₂ représente un groupe alkyle en C₁₋₆ et Z₁ représente un atome d'hydrogène ou d'halogène.

10. Procédé de préparation de composés de type phtalide conformes à la revendication 1, **caractérisé** en ce que l'on fait réagir un composé de type 2-méthylènepyrroline, de formule 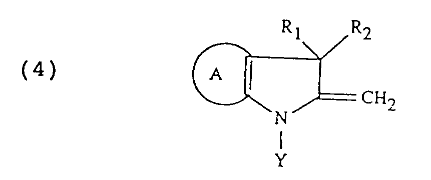 dans laquelle A, R₁, R₂ et Y ont les significations indiquées dans la revendication 1, avec un aldéhyde-acide ou un céto-acide de formule 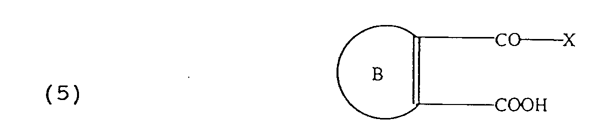 dans laquelle X et B ont les significations indiquées dans la revendication 1.

11. Matériau d'enregistrement sensible à la chaleur ou à la pression, **caractérisé** en ce qu'il contient, dans son système de réactifs chromogènes, au moins un composé de type phtalide correspondant à la formule (1), conforme à la revendication 1, en tant qu'agent chromogène, y compris le composé 3-[(1,3-dihydro-1,3,3-triméthyl-2H-indole-2-ylidène)méthyl]-1(3H)-isobenzofuranone.

12. Matériau d'enregistrement sensible à la pression, conforme à la revendication 11, **caractérisé** en ce qu'il contient du composé de type phtalide à l'état dissous dans un solvant organique, ainsi qu'au moins un compose solide accepteur d'électrons.

13. Matériau d'enregistrement sensible à la pression, conforme à l'une des revendications 11 et 12, **caractérisé** en ce que le composé de type phtalide est enfermé dans des microcapsules.

14. Matériau d'enregistrement sensible à la pression, conforme à la revendication 13, **caractérisé** en ce que le composé encapsulé de type phtalide s'y trouve sous la forme d'une couche étalée au verso d'une feuille de transfert, et l'accepteur d'électrons s'y trouve sous la forme d'une couche étalée au recto d'une feuille réceptrice.

15. Matériau d'enregistrement sensible à la chaleur, conforme à la revendication 11, **caractérisé** en ce qu'il contient, au sein d'au moins une couche, au moins un composé de type phtalide, selon la revendication 11, un accepteur d'électrons, et éventuellement, un liant et/ou une cire.

16. Matériau d'enregistrement conforme à l'une des revendications 11 à 15, **caractérisé** en ce qu'il contient, conjointement avec le composé de type phtalide, un ou plusieurs autres agents chromogènes.
